Europäisches Patentamt

**European Patent Office**

Office européen des brevets

(11) Publication number: **0 104 732**
**A1**

# (12) EUROPEAN PATENT APPLICATION

(21) Application number: **83304523.0**

(22) Date of filing: **04.08.83**

(51) Int. Cl.³: **A 61 B 17/14**
**A 61 F 5/00**

(30) Priority: **10.09.82 CA 411162**

(43) Date of publication of application:
**04.04.84 Bulletin 84/14**

(84) Designated Contracting States:
**DE FR GB IT**

(71) Applicant: **QUEEN'S UNIVERSITY AT KINGSTON**

**Kingston Ontario K7L 3N6(CA)**

(72) Inventor: **Cooke, Theodore Derek Vernon**
**728 Holgate Crescent**
**Kingston Ontario(CA)**

(72) Inventor: **Saunders, Gerald**
**R.R. 1, Sydenham**
**Ontario(CA)**

(74) Representative: **Wood, Anthony Charles et al,**
**c/o MICHAEL BURNSIDE & PARTNERS 2 Serjeants' Inn**
**Fleet Street**
**London EC4Y 1HL(GB)**

(54) **Orthopedic bone cutting device.**

(57) A slide bed device for mounting an orthopedic bone cutting device so as to provide a surgeon with three accurately controlled degrees of freedom of movement of the cutter thereby facilitating extremely accurate placement of the saw preparatory to making in vivo bone cuts. The cutting device (25) is slidably mounted (20) on a vertical stand (18) for movement in a vertical plane, and the vertical stand (18) is in turn mounted perpendicularly on two relatively movable horizontal slides (15,17) at right angles to each other. The horizontal slides (15,17) are mounted on a three point frame (6,11,12) arranged for rigid adjustable mounting on the bone to be cut in a plane parallel the longitudinal axis of the bone.

FIG. 5

## Orthopedic Bone Cutting Device          0104732

This invention relates to a device to facilitate accurate in vivo bone cuts using an orthopedic bone cutting device. More particularly this invention relates to the provision of a slide bed device for mouting an orthopedic bone cutting device, such as a saw in such a manner that the surgeon has three accurately controlled degrees of freedom of movement thereby facilitating extremely accurate placement of the cutter.

In order to insert prosthetic devices, such as partial or total knee replacements, it is, of course, necessary for the surgeon to remove sufficient bone to make room for the device, and to prepare the cut ends of the bone to receive and anchor the prosthetic device. Most prosthetic devices are manufactured to extremely precise tolerances and precise implantation in the body is necessary as inaccurately placed prostheses do not work well and loosen more easily. It is, therefore, of critical importance that the bone should be cut accurately. Heretofore this has been attempted by using a variety of jigs which stabilize the cutting blade in a predetermined direction while the saw is held in the surgeon's hand. Each cut requires a different jig as does each design of prosthesis, of which there are many. Furthermore, the jigs themselves tend to flex the blade during cutting thus reducing the chances for an accurate square cut. Fine changes or trimming are generally done "free-hand" or eyeballed and the whole procedure may be considered analogous to a carpenter trying to square the end of a piece of wood using only hand tools as opposed to using professional machine tools.

It is, therefore, an object of the present invention to overcome the aforesaid problems by obviating the use of jigs entirely and providing in their stead a workbench which may be accurately mounted on the patient's limb and which provides accurately mounted slides to provide movement in three directions. The orthopedic saw may be mounted on the slides so that it is taken entirely out of the hands of the surgeon but can be accurately controlled by him.

Thus by one aspect of this invention there is provided a device for mounting an orthopedic bone cutting device so as to facilitate accurate in vivo bone cuts, comprising:

(a)   frame means including attachment means for adjustably mounting said frame on a bone to be cut;

(b)   first slide bed means mounted on said frame in a first plane substantially parallel the longitudinal axis of said bone;

(c)   first slide means mounted in said first slide bed means for reciprocal longitudinal movement therealong;

(d)   second slide bed means mounted on said first slide means;

(e)   second slide means mounted on said second slide bed means for reciprocal longitudinal movement, in a second plane parallel said longitudinal axis of said bone and perpendicular to the direction of movement of said first slide means;

(f)   stand means mounted on said second slide means in a plane perpendicular thereto and parallel the longitudinal axis of said first slide means;

(g)   third slide means mounted on said stand means for reciprocal longitudinal movement therealong in a plane perpendicular said first and second planes; and

(h) means to releasably secure said bone cutting device to said third slide means so that a cutting edge thereof may be moved into cutting engagement with said bone at a selected position and angle thereto.

The invention will be described in more detail hereinafter with reference to the drawings in which:-

Figure 1 is a side view of one embodiment of the frame arrangement of the present invention, mounted on the tibia adjacent the knee;

Figure 2 is a plan view of the embodiment of Figure 1;

Figure 3 is a side view of the slide arrangement according to one embodiment of the present invention showing the mounting of an orthopedic saw;

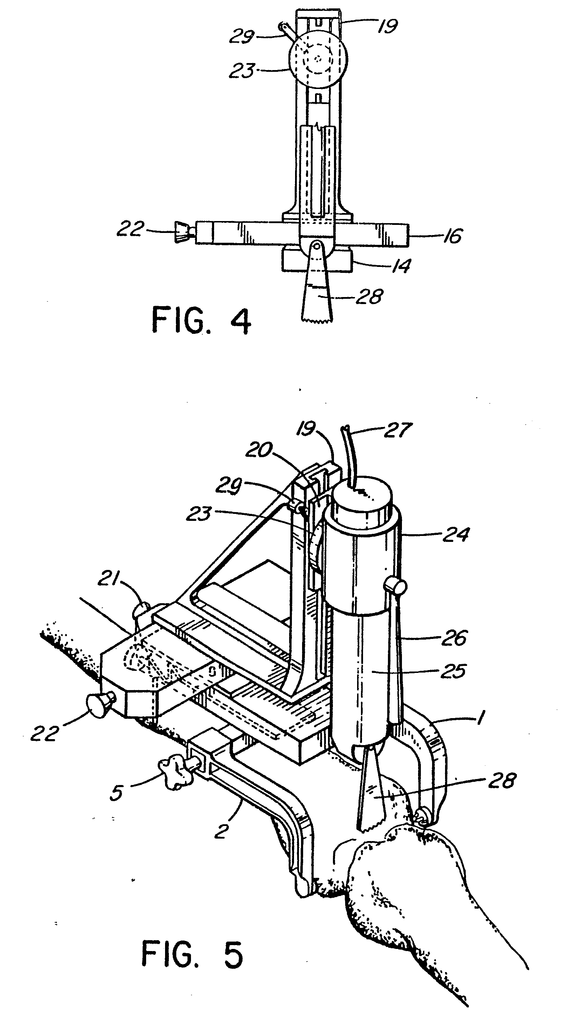

Figure 4 is a front elevational view of the embodiment of Figure 3, taken along the line 4-4 of Figure 3; and

Figure 5 is an isometric view of the frame, slide and saw arrangements mounted on the tibia.

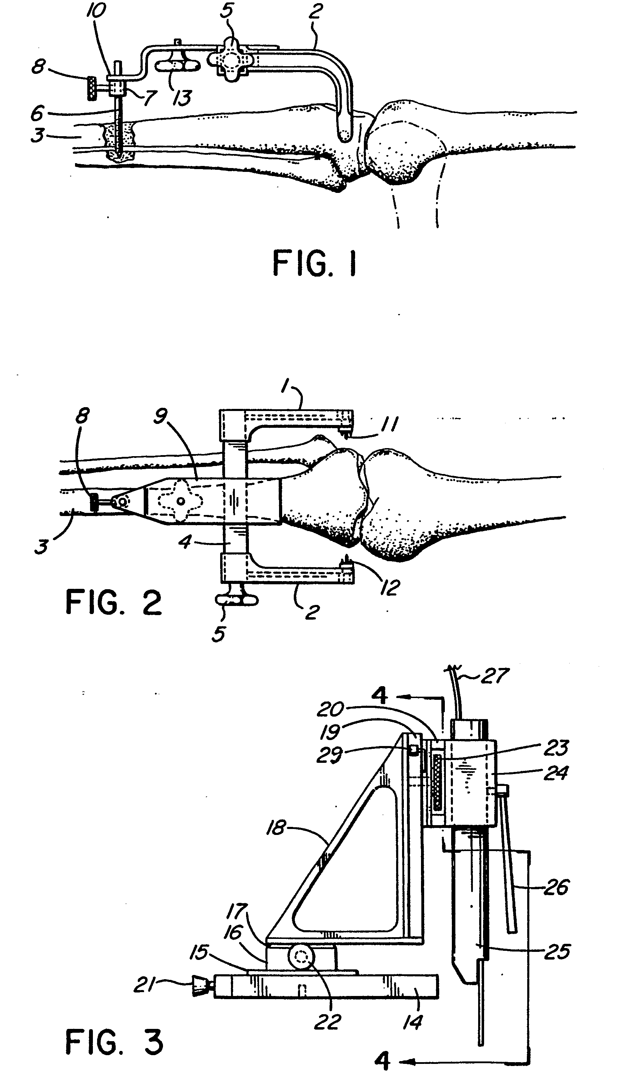

As seen in Figures 1 and 2, two L-shaped members 1,2 are arranged one on each side of a tibia 3, secured thereto by means of pins 11, 12, and adjustably and clampingly interconnected by a bar 4 provided with clamping bolt and knob 5. A pin 6 is screwed into the tibia and provided with a sleeve 7 which can be clamped thereto, by knurled clamping bolt and knob 8, at any desired position. A flat plate 9 is mounted on bar 4, intermediate L-shaped members 1,2, with one end 10 thereof slidably mounted on pin 6 and adjustably supported by sleeve 7. It will be appreciated that plate 9 is effectively pivotally mounted about pins 11, 12, at the ends of arms 1 and 2 and arranged to be inserted into the head of the tibia 3, and can be secured in any desired plane relative to the tibia 3 by simple adjustment of sleeve 7 and clamping knob 8. A mounting screw 13 is mounted through plate 9 and arranged to secure thereagainst a slide bed 14 (Figure 3). Bed 14 is provided with a slide 15 for longitudinal movement therealong. Slide 15 is provided with a second slide bed 16, mounted transversely thereto. Bed 16 in turn is provided with a slide 17 for movement transverse (i.e., at right angles) to the direction of movement of slide 15. A frame 18 is mounted on slide 17 perpendicular thereto, and carries a slide bed 19 in a plane perpendicular to the planes of beds 14 and 16. A slide 20 is mounted for longitudinal sliding movement in bed 19. It will be appreciated that slides 15, 17 and 20 may be locked in any desired or selected position in their respective beds 14, 16 and 19 by adjustment of respective clamping screws 21, 22 or lever 29 respectively.

Slide 20 is provided with a longitudinally extending sleeve member 24 arranged to receive and releasbly secure therein an orthopedic saw 25. Clamping lever 26 is provided to secure saw 25 in sleeve 24 in a conventional manner. Saw 25 may be connected, via leads or pipes 27 to an electrical power source or air apply (not shown) as required. Saw 25 is also provide with a laterally oscillating segmental blade 28.

It will also be appreciated that battery powered cutting devices, preferably with a built-in, but removable, power pack may also be employed. In a preferred embodiment the power pack may be incorporated in slide 20 and the cutting device secured

0104732

directly thereto in a conventional bayonet.

In operation, the surgeon mounts the device on the tibia 3, and adjusts the level thereof as described above. The saw 25 may be accurately positioned for cutting and clamped into that position by appropriately manipulating the three slides in each of their respective planes perpendicular to each other and securing the clamping screws in the desired position. The angle of the saw relative to the plane of frame 18 may be adjusted by clmaping screw 23 adjacent clamping lever 29.

It will, of course, be appreciated that numerous modifications may be made without departing from the scope of this invention. For example, flat segmental blade 28 may be replaced with either a rotary burr, so that a willing cut can be achieved, a vertically reciprocating blade or a curved segmental blade oscillating about the longitudinal axis thereof. Other modifications will readily suggest themselves to those skilled in the art.

6.

0104732

WE CLAIM:

1.     A device for mounting an orthopedic bone cutting device so as to facilitate accurate in vivo bone cuts, characterized by:

(a)  frame means (2) including attachment means (6, 11, 12) for adjustably mounting said frame on a bone to be cut;

(b)  first slide bed means (14) mounted on said frame in a first plane substantially parallel the longitudinal axis of said bone;

(c)  first slide means (15) mounted in said first slide bed for reciprocal longitudinal movement therealong;

(d)  second slide bed means (16) mounted on said first slide means;

(e)  second slide means (17) mounted on said second slide bed means for reciprocal longitudinal movement, in a second plane parallel said longitudinal axis of said bone, and perpendicular to the direction of movement of said first slide means;

(f)  stand means (18) mounted on said second slide means in a plane perpendicular thereto and parallel the longitudinal axis of said first slide means;

(g)  third slide means (20) mounted on said stand means for reciprocal longitudinal movement therealong in a plane perpendicular said first and second planes; and

(h)  means (26) to releasably secure said cutting device (25) to said third slide means so that a cutting edge (28) thereof may be moved into cutting engagement with said bone at a selected position and angle thereto.

0104732

2. A device as claimed in claim 1 characterized in that said attachment means comprises first pin means (6) for insertion into said bone perpendicular the longitudinal axis thereof and second and third pin means (11, 12) for insertion into respective opposite sides of said bone in a plane perpendicular to said first pin means and remote therefrom.

3. A device as claimed in claim 2 characterized in that adjusting means (8) are provided on said first pin means for adjusting said frame relative to said bone.

4. A device as claimed in claim 1, 2 or 3 characterized in that said cutting edge (28) comprises a segmental laterally oscillating saw blade.

FIG. 1

FIG. 2

FIG. 3

0104732

FIG. 4

FIG. 5

**European Patent Office**

**EUROPEAN SEARCH REPORT**

EP 83304523.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A | US - A - 2 666 430 (H.A. GISBERT) <br> * Fig. 1; column 2, lines 12-32; column 2, line 55 - column 4, line 18 * | 1 | A 61 B 17/14 <br> A 61 F 5/00 |
| A | GB - A - 2 040 168 (H. JAQUET) <br> * Fig. 2; page 3, line 97 - page 4, line 55 * | 2,3 | |
| A | US - A - 4 106 181 (T.M. MATTCHEN) <br> * Fig. 1,7; abstract * | 4 | |
| A | US - A - 4 257 411 (K. CHO) <br> * Fig.; abstract * | | |

**TECHNICAL FIELDS SEARCHED (Int. Cl. ³)**

A 61 B 17/00

A 61 F 5/00

The present search report has been drawn up for all claims

| Place of search | Date of completion of the search | Examiner |
|---|---|---|
| VIENNA | 21-12-1983 | LUDWIG |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82